# EUROPEAN PATENT SPECIFICATION

(11) **EP 3 263 137 B1**
(45) Date of publication and mention of the grant of the patent: **18.08.2021**
(21) Application number: 16194299.0
(22) Date of filing: 18.10.2016
(51) Int. Cl.: A61L 2/10, C02F 1/32

(54) **APPARATUS AND METHOD FOR STERILIZING AQUEOUS SOLUTION USING FLEXIBLE ULTRAVIOLET IRRADIATOR**
VORRICHTUNG UND VERFAHREN ZUM STERILISIEREN EINER WÄSSRIGEN LÖSUNG MIT FLEXIBLEM UV-STRAHLER
APPAREIL ET PROCÉDÉ DE STÉRILISATION DE SOLUTION AQUEUSE À L'AIDE D'UN IRRADIATEUR ULTRAVIOLET SOUPLE

(30) Priority: 29.06.2016 KR 20160081730
(43) Date of publication of application: 03.01.2018
(73) Proprietor: Korea Institute of Science and Technology, Seoul 02792 (KR)
(72) Inventor: KWON, Manjae, 02792 Seoul (KR); KIM, Hyoungseok, 02792 Seoul (KR); LEE, Taeksung, 02792 Seoul (KR); LEE, Taikjin, 02792 Seoul (KR); YANG, Jungseok, 02792 Seoul (KR); LEE, Juyoung, 02792 Seoul (KR); NHO, Chuwon, 02792 Seoul (KR); OH, Sangrok, 25451 Gangwon-do (KR); LEE, Juyeon, 25451 Gangwon-do (KR); PARK, Junwoo, 25451 Gangwon-do (KR)
(74) Representative: Walaski, Jan Filip

(56) References cited:
- WO-A1-2012/021051
- KR-B1- 100 718 676
- US-A1- 2006 131 246
- US-A1- 2012 261 319
- None

## Description

The present invention relates to an apparatus for sterilizing aqueous solution, and more particularly, to an apparatus and a method for sterilizing aqueous solution using a flexible ultraviolet irradiator, which sterilizes aqueous solution using an ultraviolet light emitting diode (LED).

Generally, to solve the shortage of water for cultivation and to maximize the usage of fertilizer ingredients contained in aqueous solution, it may be preferable to employ a method of cultivation in which aqueous solution that has been used once is circulated and reused.

Here, to recirculate and reuse the aqueous solution, a control of excessive breeding of microorganisms takes precedence and this may be performed through sterilization of the aqueous solution. However, a conventional sterilization method of aqueous solution using a chemical material such as chlorine causes a problem in that growth of plants may be impeded.

To address such a problem, a sterilization method of aqueous solution using ultraviolet rays which are used in a variety of fields has been studied, but this method also has a problem in that sterilization efficiency is varied according to an irradiation direction of the ultraviolet rays.

Therefore, there is a need for an apparatus for sterilizing aqueous solution capable of stably providing sterilization efficiency without affecting growth of plants.

US 2012/261319 A1 relates to a water sterilizing apparatus.

WO 2012/021051 relates to an apparatus for disinfecting liquids

According to an aspect of the current invention, there is provided an apparatus according to claim 1. According to another aspect of the current invention, there is provided a method according to claim 4.

The present invention is directed to providing an apparatus and a method for sterilizing aqueous solution using a flexible ultraviolet irradiator, which are capable of increasing residence time of aqueous solution to secure time required for ultraviolet sterilization.

The present invention is also directed to providing an apparatus and a method for sterilizing aqueous solution using a flexible ultraviolet irradiator, which are capable of simplifying a structure to facilitate maintenance and reduce costs related thereto.

One aspect of the present invention provides an apparatus for sterilizing aqueous solution using a flexible ultraviolet irradiator, which includes an aqueous solution flow tube formed in a helical shape, configured to block exposure of ultraviolet rays from an inside of the aqueous solution flow tube to an outside thereof, and through which aqueous solution flows; and an ultraviolet irradiator formed of a flexible material to be inserted inside the aqueous solution flow tube and configured to irradiate the aqueous solution with the ultraviolet rays to sterilize the aqueous solution.

Also, the aqueous solution flow tube includes an inlet portion configured to enable the aqueous solution to flow in; an outlet portion configured to enable the aqueous solution to flow out; a reaction portion formed in the helical shape between the inlet portion and the outlet portion and at which the aqueous solution is sterilized by the irradiation of the ultraviolet rays; and valves respectively provided between the inlet portion and the reaction portion and between the outlet portion and the reaction portion, and configured to control a flow of the aqueous solution.

Further, the ultraviolet irradiator includes a plurality of ultraviolet light emitting diodes (LEDs) configured to irradiate the aqueous solution with the ultraviolet rays; a flexible substrate connected to the plurality of ultraviolet LEDs to supply electric power thereto, inserted inside the reaction portion, and having one end formed in an annular shape; and may further include a coating layer formed outside the plurality of ultraviolet LEDs and the flexible substrate and configured to block the plurality of ultraviolet LEDs and the flexible substrate from coming into contact with the aqueous solution.

In addition, the reaction portion includes a fixing portion formed at a part in contiguity with the inlet portion and coupled to the annular shape to fix one end of the flexible substrate; and a through hole formed at a part in contiguity with the outlet portion and configured to pass through and connect the other end of the flexible substrate to an external electric power source.

Moreover, the reaction portion may be attached to and detached from the inlet portion and the outlet portion by means of the valves.

Additionally, the aqueous solution may be nutrient solution.

Another aspect of the present invention provides a method for sterilizing aqueous solution using a flexible ultraviolet irradiator according to claim 1, which includes flowing aqueous solution into an aqueous solution flow tube that is formed in a helical shape and is configured to block exposure of ultraviolet rays from an inside of the aqueous solution flow tube to an outside thereof; sterilizing the aqueous solution, which flows in the aqueous solution flow tube, using ultraviolet rays irradiated from an ultraviolet irradiator that is inserted inside the aqueous solution flow tube and is formed of a flexible material; and discharging the ultraviolet sterilized aqueous solution from the aqueous solution flow tube.

According to the present invention, an apparatus and a method for sterilizing aqueous solution may increase residence time of aqueous solution to secure time required for ultraviolet sterilization.

Also, a structure may be simplified to facilitate maintenance and reduce costs related thereto.

### BRIEF DESCRIPTION OF DRAWINGS

FIG. 1 is a diagram for describing an aqueous solution sterilizing apparatus according to one embodiment of the present disclosure.
FIG. 2 is a diagram enlarging and dissecting a region A of FIG. 1.
FIG. 3 is a flow chart for describing a method for sterilizing aqueous solution according to one embodiment of the present disclosure.

### DETAILED DESCRIPTION

Hereinafter, some embodiments of the present invention will be described in detail with reference to the accompanying drawings. In giving reference numerals to components of the drawings, the same reference numerals are given to the same components although shown in a different drawing. Also, in the following description of the present invention, if a detailed description of known functions and configurations is determined to obscure the interpretation of embodiments of the present invention, the detailed description thereof will be omitted.

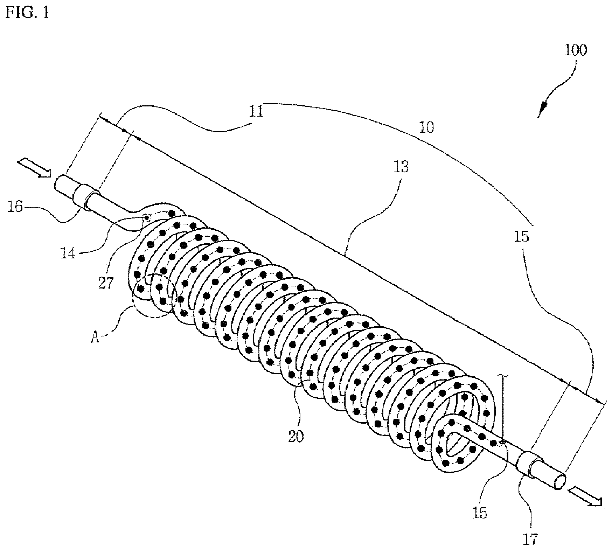

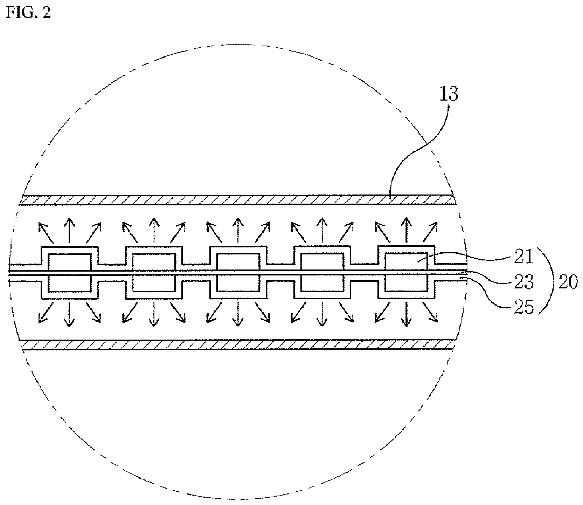

FIG. 1 is a diagram for describing an aqueous solution sterilizing apparatus according to one embodiment of the present disclosure, and FIG. 2 is a diagram enlarging and dissecting a region A of FIG. 1.

Referring to FIGS. 1 and 2, an aqueous solution sterilizing apparatus 100 increases residence time of aqueous solution to secure time required for ultraviolet sterilization. A structure of the aqueous solution sterilizing apparatus 100 may be simplified to facilitate maintenance and reduce costs related thereto. Here, the aqueous solution may be nutrient solution that is used in a hydroponics farm. The aqueous solution sterilizing apparatus 100 includes an aqueous solution flow tube 10 and an ultraviolet irradiator 20.

The aqueous solution flow tube 10 is formed to block exposure of ultraviolet rays from an inside of the aqueous solution flow tube 10 to an outside thereof, and aqueous solution flows inside the aqueous solution flow tube 10. The aqueous solution flow tube 10 is configured with an inlet portion 11, a reaction portion 12, and an outlet portion 13.

The inlet portion 11 is a section at which aqueous solution flows from the outside, the reaction portion 12 is a section at which ultraviolet rays are irradiated and thus the aqueous solution is sterilized, and the outlet portion 13 is a section at which the sterilized aqueous solution flows to the outside. At this point, the reaction portion 12 is formed in a helical shape between the inlet portion 11 and the outlet portion 13. With such a configuration, the reaction portion 12 increases an elapsed time of the aqueous solution for flowing from the inlet portion 11 to the outlet portion 13.

That is, in a conventional aqueous solution flow tube formed in a straight line shape, an elapsed time of the aqueous solution for flowing in and out is short so that a time required for ultraviolet sterilization could not secured. However, the aqueous solution flowing in the inlet portion 11 flows along a helical shape tube of the reaction portion 12 of the present invention to flow to the outlet portion 13 so that the elapsed time of the aqueous solution for flowing in and out is increased. Through such a configuration, a stay time of the aqueous solution staying inside the reaction portion 12 may be increased and this refers that a time for ultraviolet sterilization may be more secured.

The reaction portion 12 includes a fixing portion 14 and a through hole 15. The fixing portion 14 is formed at a part in contiguity with the inlet portion 11, and is fixed to one end of a flexible substrate 23, which will be described below, of the ultraviolet irradiator 20. At this point, the fixing portion 14 may be formed to include a protrusion of a rack shape and thus may be fixed to the one end of the flexible substrate 23. Preferably, the fixing portion 14 may be a hook shape, but it is not limited thereto. The through hole 15 may be formed at a part in contiguity with the outlet portion 13, and the other end of the flexible substrate 23 may pass through the through hole 15 to be connected to an external power source. That is, the through hole 15 is a hole enabling the flexible substrate 23 to be connected to the outside. At this point, an O-ring may be provided at an inner circumferential surface of the through hole 15 to prevent a gap spaced apart from the flexible substrate 23.

The aqueous solution flow tube 10 includes an inlet valve 16 and an outlet valve 17 which are provided between the inlet portion 11 and the reaction portion 12 and between the outlet portion 13 and the reaction portion 12, respectively. The inlet valve 16 and the outlet valve 17 may control a flow of aqueous solution.

For example, the inlet valve 16 may be closed to block the aqueous solution from flowing in the inlet portion 11, or may be opened to allow the aqueous solution to flow in the inlet portion 11. Also, the outlet valve 17 may be closed to block the aqueous solution, which is undergone ultraviolet sterilization in the reaction portion 12, from flowing out to the outside, or may be opened to allow the aqueous solution, which is undergone ultraviolet sterilization in the reaction portion 12, to flow out to the outside.

Also, the inlet valve 16 and the outlet valve 17 serve as media to enable the reaction portion 12 to be attached to and detached from the inlet portion 11 and the outlet portion 13. That is, the aqueous solution flow tube 10 may be configured such that the reaction portion 12 formed between the inlet valve 16 and the outlet valve 17 is separated from the aqueous solution flow tube 10 using the inlet valve 16 and the outlet valve 17and is cleaned and replaced.

The ultraviolet irradiator 20 is inserted inside the aqueous solution flow tube 10 and performs ultraviolet sterilization by irradiating aqueous solution with ultraviolet rays. In particular, the ultraviolet irradiator 20 is inserted inside the reaction portion 12 of the aqueous solution flow tube 10. Therefore, the ultraviolet irradiator 20 may have undergone a coating process to be prevented from physically coming into contact with the aqueous solution. The ultraviolet irradiator 20 includes a plurality of light emitting diodes (LEDs) 21, the flexible substrate 23, and a coating layer 25.

The plurality of LEDs 21 irradiates aqueous solution with ultraviolet rays. The plurality of LEDs 21 emit light of ultraviolet C (UV-C) having a wavelength in the range of 100 to 280 nm. At this point, the plurality of LEDs 21 may induce spontaneous generation of ozone O₃ when a wavelength is equal to or less than 200 nm, and may increase sterilization efficiency using the generated ozone. Preferably, the plurality of LEDs 21 may obtain optimal sterilization and deodorization effects at a wavelength of 184.9 nm, that is, an ozone forming range.

The flexible substrate 23 is connected to the plurality of LEDs 21 and supplies electric power thereto. At this point, the flexible substrate 23 may be connected to the plurality of LEDs 21 which are attached to one surface of the flexible substrate 23, or to the plurality of LEDs 21 which are attached to both surfaces of the flexible substrate 23. Preferably, the flexible substrate 23 may be connected to the plurality of LEDs 21 which are attached to the both surfaces of the flexible substrate 23 to enable the plurality of LEDs 21 to irradiate ultraviolet rays in all directions. The flexible substrate 23 may be fine and flexible to be inserted into along an inside of the reaction portion 12. One end of the flexible substrate 23 is formed in an annular shape 27 and is coupled and fixed to the fixing portion 14 of the aqueous solution flow tube 10, and the other end of the flexible substrate 23 passes through the through hole 15 of the aqueous solution flow tube 10 to be connected to the external power source.

The coating layer 25 is formed outside the plurality of LEDs 21 and the flexible substrate 23 and blocks them from coming into contact with the aqueous solution. That is, the coating layer 25 prevents generation of a short circuit due to a contact of the plurality of LEDs 21 and the flexible substrate 23 with the aqueous solution. The coating layer 25 may be formed of a flexible material.

As described above, the aqueous solution sterilizing apparatus 100 may directly perform ultraviolet sterilization on the aqueous solution using the ultraviolet rays irradiated from the plurality of LEDs 21. That is, the aqueous solution sterilizing apparatus 100 may directly irradiate the aqueous solution with the ultraviolet rays because the plurality of LEDs 21 are inserted inside the reaction portion 12. Therefore, the aqueous solution sterilizing apparatus 100 may rapidly irradiate strong ultraviolet rays to maximize sterilization efficiency.

Also, the aqueous solution sterilizing apparatus 100 is configured with a simplified structure in which the ultraviolet irradiator 20 is inserted inside the aqueous solution flow tube 10, so that there are effects in that a size of the apparatus may be minimized and a usage amount of components may be minimized to reduce manufacturing costs.

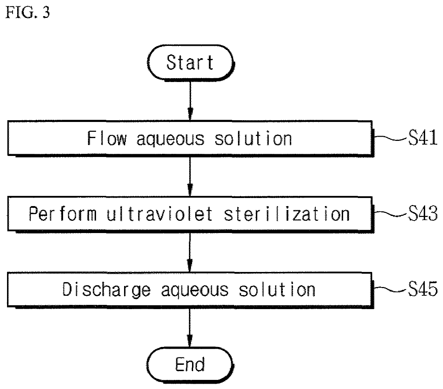

FIG. 3 is a flow chart for describing a method for sterilizing aqueous solution according to one embodiment of the present disclosure.

Referring to FIGS. 1 to 3, the method for sterilizing aqueous solution may directly irradiate ultraviolet rays to perform ultraviolet sterilization on aqueous solution so that sterilization efficiency may be maximized for a short time.

In Operation S41, aqueous solution flows in the aqueous solution sterilizing apparatus 100 through an inside of the inlet portion 11 of the aqueous solution flow tube 10. At this point, the aqueous solution may be nutrient solution used in a hydroponics farm. Here, the aqueous solution flow tube 10 is formed in a helical shape and blocks exposure of ultraviolet rays from an inside of the aqueous solution flow tube 10 to an outside thereof.

In Operation S43, the aqueous solution sterilizing apparatus 100 performs ultraviolet sterilization on the aqueous solution which flows therein. The aqueous solution sterilizing apparatus 100 performs the ultraviolet sterilization on the aqueous solution flowing through the reaction portion 12 of the aqueous solution flow tube 10 using ultraviolet rays irradiated from the plurality of LEDs 21 that are included in the ultraviolet irradiator 20. The plurality of LEDs 21 are inserted inside the reaction portion 12 such that the aqueous solution sterilizing apparatus 100 directly irradiates the aqueous solution with the ultraviolet rays.

In Operation S45, the aqueous solution sterilizing apparatus 100 discharges the ultraviolet sterilized aqueous solution. The aqueous solution sterilizing apparatus 100 discharges the ultraviolet sterilized aqueous solution to the outside through the outlet portion 13 of the aqueous solution flow tube 10. At this point, the discharged aqueous solution may be provided again to and reused in the hydroponics farm, or may be drained to the outside.

As described above, although the present invention has been described by way of a preferred embodiment and the accompanying drawings, it is not limited thereto. The invention is defined by the appended claims. Any information falling outside the scope of the claims is for explanation only.

### [Description of Reference Numerals]

| | | | |
|---|---|---|---|
| 10: | Aqueous Solution Flow Tube | 11: | Inlet Portion |
| 12: | Reaction Portion | 13: | Outlet Portion |
| 14: | Fixing Portion | 15: | Through Hole |
| 16: | Inlet Valve | 17: | Outlet Valve |
| 20: | Ultraviolet Irradiator | | |
| 21: | Ultraviolet Light Emitting Diode (LED) | | |
| 23: | Flexible Substrate | 25: | Coating layer |
| 27: | Annular Shape | | |
| 100: | Aqueous Solution Sterilizing Apparatus | | |

## Claims

1. An apparatus for sterilizing aqueous solution using a flexible ultraviolet irradiator (20), comprising:
an aqueous solution flow tube (10) formed in a helical shape, configured to block exposure of ultraviolet rays from an inside of the aqueous solution flow tube to an outside thereof, and through which aqueous solution flows; and
an ultraviolet irradiator (20) formed of a flexible material insertable inside the aqueous solution flow tube and configured to irradiate the aqueous solution with the ultraviolet rays to sterilize the aqueous solution,
wherein the aqueous solution flow tube includes:
an inlet portion (11) configured to enable the aqueous solution to flow in;
an outlet portion (13) configured to enable the aqueous solution to flow out;
a reaction portion (12) formed in the helical shape between the inlet portion and the outlet portion and at which the aqueous solution is sterilized by the irradiation of the ultraviolet rays; and
valves (16, 17) respectively provided between the inlet portion and the reaction portion and between the outlet portion and the reaction portion, and configured to control a flow of the aqueous solution,
wherein the ultraviolet irradiator includes:
a plurality of ultraviolet light emitting diodes, LEDs,(2i) configured to irradiate the aqueous solution with the ultraviolet rays; and
a flexible substrate (23) connected to the plurality of ultraviolet LEDs to supply electric power thereto, inserted inside the reaction portion, and having one end formed in an annular shape, and
wherein the reaction portion includes:
a fixing portion (14) formed at a part in contiguity with the inlet portion and coupled to the annular shape to fix one end of the flexible substrate; and
a through hole (15) formed at a part in contiguity with the outlet portion and configured to pass through and connect the other end of the flexible substrate to an external electric power source.

2. The apparatus of claim 1, wherein the ultraviolet irradiator further includes:
a coating layer (25) formed outside the plurality of ultraviolet LEDs and the flexible substrate and configured to block the plurality of ultraviolet LEDs and the flexible substrate from coming into contact with the aqueous solution.

3. The apparatus of claim 1 or 2, wherein the valves are configured to enable the reaction portion to be attached to and detached from the inlet portion and the outlet portion.

4. A method for sterilizing aqueous solution using a flexible ultraviolet irradiator, comprising:
flowing aqueous solution into an aqueous solution flow tube that is formed in a helical shape and is configured to block exposure of ultraviolet rays from an inside of the aqueous solution flow tube to an outside thereof (S₄₁);
sterilizing the aqueous solution, which flows in the aqueous solution flow tube, using ultraviolet rays irradiated from an ultraviolet irradiator that is inserted inside the aqueous solution flow tube and is formed of a flexible material (S₄₃); and
discharging the ultraviolet sterilized aqueous solution from the aqueous solution flow tube (S₄₅),
wherein the aqueous solution flow tube includes:
an inlet portion (11) configured to enable the aqueous solution to flow in;
an outlet portion (13) configured to enable the aqueous solution to flow out;
a reaction portion (12) formed in the helical shape between the inlet portion and the outlet portion and at which the aqueous solution is sterilized by the irradiation of the ultraviolet rays; and
valves (16, 17) respectively provided between the inlet portion and the reaction portion and between the outlet portion and the reaction portion, and configured to control a flow of the aqueous solution,
wherein the ultraviolet irradiator includes:
a plurality of ultraviolet light emitting diodes, LEDs,(21) configured to irradiate the aqueous solution with the ultraviolet rays; and
a flexible substrate (23) connected to the plurality of ultraviolet LEDs to supply electric power thereto, inserted inside the reaction portion, and having one end formed in an annular shape, and
wherein the reaction portion includes:
a fixing portion (14) formed at a part in contiguity with the inlet portion and coupled to the annular shape to fix one end of the flexible substrate; and
a through hole (15) formed at a part in contiguity with the outlet portion and configured to pass through and connect the other end of the flexible substrate to an external electric power source.

5. The method of claim 4, wherein the aqueous solution is nutrient solution.

## Patentansprüche

1. Vorrichtung zum Sterilisieren einer wässrigen Lösung mit einem flexiblen UV-Strahler (20), die Folgendes umfasst:
eine spiralförmig ausgebildete Durchflussröhre (10) für wässrige Lösung, konfiguriert zum Verhindern, dass UV-Strahlen von einer Innenseite des Durchflussrohrs für wässrige Lösung zu einer Außenseite davon gelangen, und durch das wässrige Lösung fließt; und
einen aus einem flexiblen Material gebildeten UV-Strahler (20), der in das Durchflussrohr für wässrige Lösungen einführbar und zum Bestrahlen der wässrigen Lösung mit den UV-Strahlen zum Sterilisieren der wässrigen Lösung konfiguriert ist,
wobei das Durchflussrohr für wässrige Lösung Folgendes beinhaltet:
einen Einlassabschnitt (11), der so konfiguriert ist, dass die wässrige Lösung einströmen kann;
einen Auslassabschnitt (13), der so konfiguriert ist, dass die wässrige Lösung ausströmen kann;
einen Reaktionsabschnitt (12), der zwischen dem Einlassabschnitt und dem Auslassabschnitt spiralförmig ausgebildet ist und in dem die wässrige Lösung durch Bestrahlen mit den UV-Strahlen sterilisiert wird; und
Ventile (16, 17), die jeweils zwischen dem Einlassabschnitt und dem Reaktionsabschnitt und zwischen dem Auslassabschnitt und dem Reaktionsabschnitt vorgesehen und so konfiguriert sind, dass sie einen Fluss der wässrigen Lösung steuern,
wobei der UV-Strahler Folgendes umfasst:
mehrere UV-Leuchtdioden, LEDs, (21), konfiguriert zum Bestrahlen der wässrigen Lösung mit den UV-Strahlen; und
ein flexibles Substrat (23), das mit den mehreren UV-LEDs verbunden ist, um diese mit elektrischer Energie zu versorgen, das in den Reaktionsabschnitt eingeführt ist und von dem ein Ende ringförmig ausgebildet ist, und
wobei der Reaktionsabschnitt Folgendes beinhaltet:
einen Befestigungsabschnitt (14), der an einem Teil angrenzend an den Einlassabschnitt ausgebildet und mit der Ringform gekoppelt ist, um ein Ende des flexiblen Substrats zu fixieren; und
ein Durchgangsloch (15), das an einem Teil angrenzend an den Auslassabschnitt ausgebildet und so konfiguriert ist, dass es durch das andere Ende des flexiblen Substrats geht und es mit einer externen elektrischen Energiequelle verbindet.

2. Vorrichtung nach Anspruch 1, wobei der UV-Strahler ferner Folgendes aufweist:
eine Überzugsschicht (25), die außerhalb der mehreren UV-LEDs und des flexiblen Substrats ausgebildet und zum Verhindern eines Kontakts der mehreren UV-LEDs und des flexiblen Substrats mit der wässrigen Lösung konfiguriert ist.

3. Vorrichtung nach Anspruch 1 oder 2, wobei die Ventile so konfiguriert sind, dass der Reaktionsabschnitt am Einlassabschnitt und am Auslassabschnitt angebracht und davon abgenommen werden kann.

4. Verfahren zum Sterilisieren einer wässrigen Lösung mit einem flexiblen UV-Strahler, das Folgendes beinhaltet:
Leiten von wässriger Lösung in ein spiralförmig ausgebildetes Durchflussrohr für wässrige Lösung, konfiguriert zum Verhindern, dass UV-Strahlen von einer Innenseite des Durchflussrohrs für wässrige Lösung zu einer Außenseite davon (S41) gelangen;
Sterilisieren der im Durchflussrohr für wässrige Lösung fließenden wässrigen Lösung mit UV-Strahlen, die von einem UV-Strahler ausgestrahlt werden, der in das Durchflussrohr für wässrige Lösung eingesetzt und aus einem flexiblen Material gebildet ist (S43); und
Ablassen der UV-sterilisierten wässrigen Lösung aus dem Durchflussrohr für wässrige Lösung (S45),
wobei das Durchflussrohr für wässrige Lösung Folgendes beinhaltet:
einen Einlassabschnitt (11), der so konfiguriert ist, dass die wässrige Lösung einströmen kann;
einen Auslassabschnitt (13), der so konfiguriert ist, dass die wässrige Lösung ausströmen kann;
einen Reaktionsabschnitt (12), der zwischen dem Einlassabschnitt und dem Auslassabschnitt spiralförmig ausgebildet ist und in dem die wässrige Lösung durch Bestrahlen mit den UV-Strahlen sterilisiert wird; und
Ventile (16, 17), die jeweils zwischen dem Einlassabschnitt und dem Reaktionsabschnitt und zwischen dem Auslassabschnitt und dem Reaktionsabschnitt vorgesehen und zum Steuern eines Flusses der wässrigen Lösung konfiguriert sind,
wobei der UV-Strahler Folgendes umfasst:
mehrere UV-Leuchtdioden, LEDs, (21), konfiguriert zum Bestrahlen der wässrigen Lösung mit den UV-Strahlen; und
ein flexibles Substrat (23), das mit den mehreren UV-LEDs verbunden ist, um diese mit elektrischer Energie zu versorgen, das in den Reaktionsabschnitt eingesetzt ist und von dem ein Ende ringförmig ausgebildet ist, und
wobei der Reaktionsabschnitt Folgendes beinhaltet:
einen Befestigungsabschnitt (14), der an einem Teil angrenzend an den Einlassabschnitt ausgebildet und mit der Ringform gekoppelt ist, um ein Ende des flexiblen Substrats zu fixieren; und
ein Durchgangsloch (15), das an einem Teil angrenzend an den Auslassabschnitt ausgebildet und so konfiguriert ist, dass es durch das andere Ende des flexiblen Substrats geht und es mit einer externen elektrischen Energiequelle verbindet.

5. Verfahren nach Anspruch 4, wobei die wässrige Lösung eine Nährlösung ist.

## Revendications

1. Appareil de stérilisation d'une solution aqueuse à l'aide d'un irradiateur à ultraviolets souple (20), comprenant :
un tube d'écoulement de solution aqueuse (10) formé en une forme hélicoïdale, configuré pour bloquer l'exposition aux rayons ultraviolets depuis un espace interne du tube d'écoulement de solution aqueuse jusqu'à un espace externe de celui-ci, et à travers lequel coule une solution aqueuse ; et
un irradiateur à ultraviolets (20) formé en un matériau souple apte à être inséré dans un espace interne du tube d'écoulement de solution aqueuse et configuré pour irradier la solution aqueuse avec les rayons ultraviolets afin de stériliser la solution aqueuse,
dans lequel le tube d'écoulement de solution aqueuse inclut :
une portion d'entrée (11) configurée pour permettre à la solution aqueuse d'entrer par écoulement ;
une portion de sortie (13) configurée pour permettre à la solution aqueuse de sortir par écoulement ;
une portion de réaction (12) formée dans la forme hélicoïdale entre la portion d'entrée et la portion de sortie et au niveau de laquelle la solution aqueuse est stérilisée par l'irradiation des rayons ultraviolets ; et
des soupapes (16, 17) prévues respectivement entre la portion d'entrée et la portion de réaction et entre la portion de sortie et la portion de réaction, et configurées pour réguler un flux de la solution aqueuse,
dans lequel l'irradiateur à ultraviolets inclut :
une pluralité de diodes électroluminescentes, DEL, à ultraviolets (21) configurées pour irradier la solution aqueuse avec les rayons ultraviolets ; et
un substrat souple (23) connecté à la pluralité de DEL à ultraviolets afin de fournir de l'énergie électrique à celles-ci, inséré dans l'espace interne de la portion de réaction, et ayant une extrémité formée en une forme annulaire, et
dans lequel la portion de réaction inclut :
une portion de fixation (14) formée au niveau d'une partie en contiguïté avec la portion d'entrée et couplée à la forme annulaire afin de fixer une extrémité du substrat souple ; et
un trou traversant (15) formé au niveau d'une partie en contiguïté avec la portion de sortie et configuré pour traverser et connecter l'autre extrémité du substrat souple à une source d'énergie électrique externe.

2. Appareil de la revendication 1, dans lequel l'irradiateur à ultraviolets inclut en outre :
une couche de revêtement (25) formée à l'extérieur de la pluralité de DEL à ultraviolets et du substrat souple et configurée pour empêcher que la pluralité de DEL à ultraviolets et le substrat souple n'entrent en contact avec la solution aqueuse.

3. Appareil de la revendication 1 ou 2, dans lequel les soupapes sont configurées pour permettre à la portion de réaction d'être attachée à et d'être détachée de la portion d'entrée et de la portion de sortie.

4. Procédé pour stériliser une solution aqueuse à l'aide d'un irradiateur à ultraviolets souple, comprenant :
le fait de faire couler une solution aqueuse dans un tube d'écoulement de solution aqueuse qui est formé en une forme hélicoïdale et est configuré pour bloquer l'exposition aux rayons ultraviolets depuis un espace interne du tube d'écoulement de solution aqueuse jusqu'à un espace externe de celui-ci (S41) ;
le fait de stériliser la solution aqueuse, qui coule dans le tube d'écoulement de solution aqueuse, grâce à l'utilisation de rayons ultraviolets irradiés à partir d'un irradiateur à ultraviolets qui est inséré dans l'espace interne du tube d'écoulement de solution aqueuse et est formé en un matériau souple (S43) ; et
le fait de décharger la solution aqueuse stérilisée par ultraviolets à partir du tube d'écoulement de solution aqueuse (S45),
dans lequel le tube d'écoulement de solution aqueuse inclut :
une portion d'entrée (11) configurée pour permettre à la solution aqueuse d'entrer par écoulement ;
une portion de sortie (13) configurée pour permettre à la solution aqueuse de sortir par écoulement ;
une portion de réaction (12) formée dans la forme hélicoïdale entre la portion d'entrée et la portion de sortie et au niveau de laquelle la solution aqueuse est stérilisée par l'irradiation des rayons ultraviolets ; et
des soupapes (16, 17) prévues respectivement entre la portion d'entrée et la portion de réaction et entre la portion de sortie et la portion de réaction, et configurées pour réguler un flux de la solution aqueuse,
dans lequel l'irradiateur à ultraviolets inclut :
une pluralité de diodes électroluminescentes, DEL, à ultraviolets (21) configurées pour irradier la solution aqueuse avec les rayons ultraviolets ; et
un substrat souple (23) connecté à la pluralité de DEL à ultraviolets afin de fournir de l'énergie électrique à celles-ci, inséré dans l'espace interne de la portion de réaction, et ayant une extrémité formée en une forme annulaire, et
dans lequel la portion de réaction inclut :
une portion de fixation (14) formée au niveau d'une partie en contiguïté avec la portion d'entrée et couplée à la forme annulaire afin de fixer une extrémité du substrat souple ; et
un trou traversant (15) formé au niveau d'une partie en contiguïté avec la portion de sortie et configuré pour traverser et connecter l'autre extrémité du substrat souple à une source d'énergie électrique externe.

5. Procédé de la revendication 4, dans lequel la solution aqueuse est une solution de nutriment.
